# EUROPEAN PATENT SPECIFICATION

(11) **EP 0 846 169 B1**
(45) Date of publication and mention of the grant of the patent: **19.03.2003**
(21) Application number: 96926042.1
(22) Date of filing: 31.07.1996
(51) Int. Cl.: C12N 15/06, C07K 16/24, C07K 16/28, C12N 5/20, C12P 21/08, A61K 39/395, G01N 33/577, C07K 14/525, C07K 14/15

(54) **MONOCLONAL ANTIBODIES AGAINST SOLUBLE TNF-ALPHA RECEPTORS p55 and p75 AS WELL AS AGAINST TNF-ALPHA AND ITS ANALOGUES**
MONOKLONALE ANTIKÖRPER GEGEN LÖSLICHE TNF-ALPHA REZEPTOREN P55 UND P75 ALS AUCH GEGEN TNF-ALPHA UND DESSEN ANALOGEN
ANTICORPS MONOCLONAUX DIRIGES CONTRE LES RECEPTEURS SOLUBLES p55 ET p75 DU FACTEUR ALPHA DE NECROSE TUMORALE TNF-ALPHA DE MEME QUE CONTRE TNF-ALPHA ET SES ANALOGUES

(30) Priority: 04.08.1995 SI 9500249; 28.06.1996 SI 9600206
(43) Date of publication of application: 10.06.1998
(73) Proprietor: Lek, Tovarna Farmacevtskih in Kemicnih Izdelkov, D.D., 1526 Ljubljana (SI); Zavod Republike Slovenije Za Transfuzijo Krvi, 1000 Ljubljana (SI)
(72) Inventor: CURIN SERBEC, Vladka, 1000 Ljubljana (SI); SCHUURBIERS, Annette, NL-1921 XK Akersloot (NL); MENART, Viktor, 1370 Logatec (SI); GADERC POREKAR, Vladimira, 1000 Ljubljana (SI); STALC, Anton, 1000 Ljubljana (SI); ZUNEC, Petra, 9251 Tis ina (SI); OCVIRK, Janja, 1000 Ljubljana (SI); STABUC, Borut, 1000 Ljubljana (SI)
(74) Representative: Perrey, Ralf, Dr. Dipl.-Chem.
(86) International application number: SI9600018
(87) International publication number: WO97006251

(56) References cited:
- EP-A- 0 218 868
- EP-A- 0 288 088
- EP-A- 0 350 690
- EP-A- 0 492 448
- WO-A-92/22666

## Description

The present invention belongs to the field of pharmaceutical industry and relates to a series of novel monoclonal antibodies and fragments thereof which are bound either to the available surface of the human recombinant TNF-α or TNF-α analogues or TNF-α soluble receptors p55 and p75 as well as complexes thereof. Further, the invention relates to stable hybridoma cell lines, which are capable of producing such monoclonal antibodies, to a process for the preparation thereof and to pharmaceutical preparations containing them.

The invention also relates to a method for quantitative determination of human TNF-α, free or complexed with the soluble receptors, and of its analogues or soluble TNF-α receptors p55 or p75, free and complexed with TNF-α, in biological samples and to a kit used therefor. Another object of the invention is also a TNF-α analogue, i.e. TNF-α Cys95His107His108Cys148, which is used in a complex with TNF-α soluble receptors p55 and/or p75 as an antigen for the immunization of mice.

### Technical Problem

By hitherto disclosed monoclonal antibodies and reagents developed therefrom, it has not been possible to simultaneously determine free macromolecules of TNF-α or soluble receptors thereof, and complexes thereof. This determination is very important from the view of assessment of the state of disease or of the success of treatment since by the development of a disease in many cases also the level of soluble TNF-α receptors is increased, which bind TNF-α and thus lower the actual content of both receptors and TNF-α.

### Prior Art

Tumour necrosis factor alpha (TNF-α) is a cytokine which is primarily produced by monocytes and macrophages as a response to stimulation with endotoxine or other stimuli. Soluble TNF-α may be produced also by other cells: granulocytes, lymphocytes T and B as well as NK cells. Soluble TNF-α is in a trimeric form; the molecular weight of subunits is 17 kDa and the molecular weight when incorporated into the membrane is 26 kDA. A review over TNF-α has been given in several periodicals, e.g. B. Beutler et al., Nature 320, 584, 1986, and G. Ser 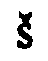 a, Zdrav. vestn. 63, Suppl. II, 35, 1994.

It was originally intended to use TNF-α in the treatment of cancerous diseases due to its antitumour activity. Hovewer, later investigations showed that TNF-α is a pluripotent and pleiotropic cytokine: its action is also expressed in e.g. decreased lipase activity, inhibition of viral propagation, activation and chemotaxis of neutrophiles, cell growth - proliferation and differentiation - of different cells, increased production of prostaglandine E2 and collagenase, decreased collagen synthesis, procoagulant activity, contractility inhibition and increased expression of the major histocompatibility complex (MHC) of classes I and II. Numerous negative effects of TNF-α are connected with the development of various severe pathological conditions such as e.g. meningitis, septic shock and other bacterial, viral or fungal infections, various acute and chronical inflammations, tissue necrosis, neoplasma, cachexia, autoimmune diseases etc. (e.g. LE Junming et al., WO 92/16553; G. Ser 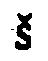 a, Zdrav. vestn. 63, Suppl. II, 35, 1994).

From the therapeutic point of view, besides the preparation of TNF-α or its less harmful analogues, also the development of products neutralizing the action of TNF-α is interesting. For instance, specific monoclonal antibodies offer such a possibility. At various diseases specific monoclonal antibodies also make possible the determination of TNF-α in some body fluids and thus significantly contribute to diagnosing various serious diseases.

The TNF-α action is achieved via receptors on different cells. There are two kinds of receptors, with molecular weights of 55 kDa and of 75 kDa (M. Brockhaus et al., Proc. Natl. Acad. Sci. USA 87, 3127, 1990). The extracellular domains of the receptors are similar (J. Vil 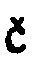 ek and TH Lee, J. Biol. Chem. 166, 7313, 1991), whereas their intracellular parts are different. This probably allows to mediate different stimulation in the cell (Z. Dembic at al., Cytokine 2, 231, 1990).

Cleaved extracellular domains of the receptors present in serum and plasma are called soluble receptors of TNF-α (P. Seckinger et al., Proc. Natl. Acad. Sci USA 87, 5188, 1990). Sometimes these proteins are called inhibitory peptides for TNF (US 5,359,037). At some diseases e.g. the amount of soluble receptors depends upon the intensity of the disease and the treatment thereof (I. Olson et al., Eur. Cytokine Netw. 4, 169, 1993), whereas the kinetics of TNF-α and thus its effectiveness probably depend upon the amount thereof in the serum (Kus et al., Int. J. Cancer 55,110, 1993). Thus, the determination of soluble receptors for TNF-α is important both from the viewpoint of diagnosing the severity of the disease as well as determining the effectiveness and expediency of the treatment. Soluble receptors for TNF-α may be determined with specific monoclonal antibodies.

Antibodies against a selected antigen may be prepared by immunizing animals with the antigen and isolating the antibodies formed from the animal serum. The antibodies so obtained are heterogenous - polyclonal; namely, they have different affinities to the antigen and they are also bound to various antigen determinants - epitopes.

Koehler and Millstein (Nature 256, 495, 1975) developed a method for the hybridization of cells, which makes possible the production of monoclonal antibodies i.e. antibodies with equal structure which are all bound to the same site and react with only one antigen determinant. Such cells - hybridomas - are formed by the fusion of lymphoblasts and myeloma cells. This method makes possible an unlimited synthesis of equal monoclonal antibodies; due to this property monoclonal antibodies are indispensable in many fields, in therapy and in diagnostics.

In EP 212489 Cerami et al. disclosed a modulatormaterial isolated from macrophages. Numerous diagnostic and therapeutic utilities are proposed, and resting procedures, materials in kit form and pharmaceutical compositions are likewise set forth. Rubin et al. developed hybridomas for producing monoclonal antibodies, and antibodies for the determination of TNF-α and the purification thereof (EP 218868).

A. Moeller and F. Emlig developed monoclonal antibodies with two chains of different weight, which gives to the antibodies a different neutralization ability and thus also a more specific utility (EP 260610).

In EP 288088 Yone et al. disclosed a method for the determination of TNF-α in the body fluids of patients having Kawasaki disease, with recognizability of epitopes on TNF-α sites from Gly68 to Ile97. These monoclonal antibodies may neutralize the cytotoxic effects of TNF-α on L9929 cells, which effects regulate the metabolism of fats and the binding to the TNF-α receptor. They also disclosed monoclonal antibodies recognizing-sites on TNF-α from Thr7 to Leu37 as well as sites from Pro113 to Glu127.

In EP 492448 E. Barbanti disclosed monoclonal antibodies or fragments thereof for the neutralization of TNF-α in the molar ratio of 2:1 and also for the detection of human TNF-α in body fluids.

In WO 92/16553 Junming et al., besides common monoclonal antibodies against TNF-α, also disclosed chimeric ones i.e. humanized antibodies of a different structure having a high affinity to TNF-α and a high neutralization ability for TNF-α; in these cases monoclonal antibodies may be bound to amino acid residues of recombinant TNF-α 59-80 and 87-108, whereas they are not bound to parts of TNF-α which are important for binding to the receptor: 11-13, 37-42, 49-57 or 155-157. The substances are useful in the diagnostis of TNF-α and for the treatment of diseases related to increased synthesis of TNF-α.

Also in WO 92/11383 Adair et al. disclosed partially humanized antibodies against TNF-α for therapy and diagnostics. It is characteristic of these antibodies that each has three specific binding sites on a variable part of both the heavy and the light chain, which makes possible a repeated and safer parenteral application.

In US 5,223,395 an immunometric assay is disclosed for biologically active tumor necrosis factor in a biological sample, comprising forming a complex of a first labeled monoclonal antibody, biologically active TNF, and a second monoclonal antibody which can be bound to an insoluble substrate and detecting the amount of label associated with the complex.

In US 5,360,716 Y. Ohomoto et al. described the preparation of monoclonal antibodies of high specificity, which may be used in the purification of TNF-α or for its quantitative determination in biological samples. Among them there are several antibodies reacting with SDS-denaturated TNF-α.

Besides antibodies against TNF-α, also Fab fragments were developed, which, due to the binding to TNF-α, are able to prevent a septic shock (KJ Tracy et al., Nature 330, 662, 1987).

For qualitative and quantitative determination of human TNF-α in biological fluids and bacterial lysates also monoclonal antibodies bispecific for TNF-α and horse radish peroxidase are used (N. Berkova et al., DE 4104787).

In WO 91/02756 M. Kriegler disclosed antibodies of different origin preventing the cleavage of pro-TNF-α into larger or smaller fragments. By combining both kinds of antibodies, the TNF-α content in various body fluids may be determined. They may also be used for various prophylactic and therapeutic purposes in diseases related to pathological TNF-α content.

P. Boyle et al. developed monoclonal antibodies useful in diagnostics. They also bind to cell surface TNF-α and prevent TNF-α secretion by human monocyte cell line induced by LPS (EP 614984).

Polyclonal and monoclonal antibodies against TNF-α are also a basic ingredient of a reagent which makes it possible to selectively determine oligomeric TNF-α, but not presumptive biologically inactive monomers or TNF-α bound to TNF receptors (A. Corti, WO 93/06489).

Antibodies against TNF-α and its fragments, which are of different origin and neutralizing by nature, are also found in combination with different xanthine derivatives intended for the treatment of different diseases related to an increased amount of TNF-α (H. Anagnostopulos, WO 92/07585).

A combination of antibodies for TNF-α and interferon γ was suggested for the treatment of diseases related to immunological system such as autoimmune diseases and transplantate rejection (Jonker and PH Van der Meide, WO 90/10707).

Combinations of antibodies against TNF-α with different antibiotics were suggested for the treatment of bacterial meningitis (RF Hector and MS Collins, EP 585705).

A number of monoclonal antibodies specific for recombinant TNF-α has been disclosed in various literature, e.g.: CH Liang, Biochem. Biophys. Res. Comm. 137, 847, 1986; A. Meger et al., Hybridoma 6, 305, 1987; Fendly et al., Hybridoma 6, 359, 1987; TS Bringman et al., Hybridoma 6, 489, 1987; M. Hirai et al., J. Immunol. Meth. 96, 57, 1987; A Mooler et al., Cytokine 2, 162, 1990. They are used either for determining epitopes on TNF-α or for developing immune methods for the determination of TNF-α or for the isolation of TNF-α. However, none of the above citations directly refers to the area of therapy or to diagnostics *in vivo* (LE Junming et al., WO 92/16553).

Considering the development of antibodies against TNF-α having different binding ability or neutralization ability, PR Temest et al., Hybridoma 13, 183, 1994 should be mentioned. In this article such monoclonal antibodies are disclosed which are partial competitive antagonists of unmodified TNF-α antibody formed before the structural changes of TNF-α.

Monoclonal antibodies and biologically active fragments against receptors for human TNF-α, which were isolated from HL-60 cells, were developed by Brockhaus and Loetscher (EP 334165). These active ingredients serve either for the purification of membrane receptors for TNF-α or for therapeutic or diagnostic purposes.

A more detailed definition of the use of monoclonal antibodies against soluble TNF receptors is evident from WO 94/09137 (Fendley et al.). They are used for the treatment of patients with malign tumours, HIV and autoimmune diseases mediated by T cells.

In US 5,359,037 Wallash et al. disclosed polyclonal and monoclonal antibodies against a receptor for TNF-α and biologically active fragments thereof; these proteins may stimulate some effects of TNF-α on cell receptors or inhibit them. They are also useful in diagnostics for detecting endogenously formed antibodies against receptors for TNF-α and for determining the TNF-α receptor level in body fluids.

In WO 92/22666 G. Adolf disclosed monoclonal antibodies against the extracellular domain of TNF-α receptor. These antibodies are useful for the determination of soluble TNF-α receptors p55 in body fluids by means of ELISA method.

### The Inventive Solution

The formulations of the present invention differ from hitherto known monoclonal antibodies and reagents developed therefrom for determining TNF-α or soluble receptors in that they make possible the determination of free receptors p55 or p75 or TNF-α as well as the determination of the complexes of the two soluble TNF-α receptors with TNF-α or its analogues. The determination of the free and bound TNF-α soluble receptors is very important for the assessment of the state of a disease or of the success of the treatment. During the development of some diseases the level of soluble TNF-α receptors with bound TNF-α is increased. Consequently, the values representing both the level of free TNF-α receptors and of the TNF-α receptors in a complex with TNF-α are a more reliable measure either of the disease or of the therapy than the determination of the free TNF-α soluble receptors alone.

Such monoclonal antibodies are obtained by cultivating the stable hybridoma cell lines deposited under ECACC 95 101 016, 95 071 217 and 95 071 218. The stable hybridoma cell lines are prepared by immunizing mice with complexes of TNF-α analogue Cys95His107His108Cys148 with soluble TNF-α receptors p55 and/or p75.

The monoclonal antibodies representing an object of the present invention may be used in diagnostics as well as in prophylaxis and/or therapy of diseases wherein the TNF-α amount is increased. Such typical diseases are e.g. septic shock, AIDS, cerebral malaria, graft-versus-host disease, rheumatoid arthritis. Monoclonal antibodies of the present invention may be used for these purposes alone or as pharmaceutical formulations. Monoclonal antibodies in a corresponding pharmaceutical formulation may be administered parenterally: subcutaneously, intravenously, intraar terially or intramuscularly.

The monoclonal antibodies of the invention are also suitable for immune tests in a liquid phase as well as on a solid phase.

The monoclonal antibodies and fragments thereof are prepared by means of immortalizing lymphocytes B of a mouse immunized with a selected antigen. This may be done either by means of transformation with an oncogenic virus, by means of electrofusion or by means of fusion with already immortalized cell line of myelomic or lymphoblastoid cells. In most cases plasmacytomic (myelomic) cells of mammal origin are used which do not possess the enzyme hypoxanthine-guanine-phosphoribosyl-transferase (HGPRT).

Cell lines are then cloned by means of one of possible methods and are subsequently tested for the ability to produce the desired antibodies. Various tests for determining specific antibodies are well-known and the choice thereof usually depends upon the nature of the antigen itself as well as upon the desired properties of antibodies. This step is the most important one in the whole process since in this step antibodies having high affinity constants and the desired specificity are chosen.

Once a stable hybridoma cell line producing monoclonal antibodies of desired specificity and affinity has been obtained, this line represents a source of genetic material encoding the monoclonal antibody.

Genes encoding the selected monoclonal antibodies against TNF-α or its analogue TNF-α Cys95His107His108Cys148 are isolated in such a way that cDNA library is prepared from mRNA. Then the cDNA encoding immunoglobulin is isolated and further manipulated. Certain parts of nucleotide sequence may be exchanged to prepare so-called humanized antibodies (in this way their immunogenicity is reduced), which are suitable for therapeutic use. cDNA clone may be inserted into an appropriate procariotic or eucariotic expression vector and is used for a possible production on larger scale.

The term "stable cell line" means that the line is viable for a long time, in theory for an unlimited period of time, and that during this time it also produces the desired monoclonal antibody.

The term "monoclonal antibody" relates to an antibody chosen from a mixture of different antibodies. All monoclonal antibodies of the same specificity and affinity are identical except for natural mutants thereof.

Under the term "antibody" intact molecules of immunoglobulins as well as fragments thereof (Fab, F(ab')₂, Fv, scFv) against TNF-α analogue are to be understood.

The term "neutralizing" means the ability of antibodies to block cytotoxic activity of TNF-α or its analogue TNF-α Cys95His107His108Cys148 *in vitro* and/or *in vivo*.

For the preparation of monoclonal antibodies of the present invention a fusion method is used, which was first disclosed by Koehler and Millstein (Koehler, G. and Millstein, C., Nature 256: 496, (1975)). NS1 plasmacytomic line is used, which is obtained in BALB/c mice, is HGPRT⁻ and does neither produce nor secrete immunoglobulins. Such an immortalized cell line is fused with cells, of which some produce antibodies being bound to TNF-α analogue Cys95His107His108Cys148 and also to TNF-α. The cells producing antibodies against TNF-α analogue are isolated from the spleen of BALB/c mice immunized with a complex of TNF-α analogue Cys95His107His108Cys148. The TNF-α analogue Cys95His107His108Cys148 is prepared by means of recombinant DNA technology. After the fusion of both kinds of cells the presence of specific antibodies is tested in the supernatants of hybridomas formed. Such antibodies are chosen which react with TNF-α analogue or also with TNF-α itself. ELISA method (enzyme linked immunosorbent assay) is used for testing, wherein both recombinant proteins (TNF-α analogue Cys95His107His108-Cys148 and TNF-α) are used for coating microtitre plates. The selected stable cell lines are cultivated in small plastic bottles or in spinner flasks. A concentration of monoclonal antibodies up to 1.0 mg/ml may be achieved in supernatants in an appropriate medium and under appropriate cultivation of cell lines. Such monoclonal antibodies may be isolated from supernatants by means of precipitation with ammonium sulfate or by means of ion exchange chromatography or affinity chromatography on protein A- or protein G-Sepharose.

For immunization the complexes of the composition ABₙ₁, ABₙ₂ or the mixture ABₙ₁Bₙ₂ were chosen and prepared.

The meanings of above symbols are as follows:
- A: means the TNF-α analogue TNF-α Cys95His107His108Cys148,
- ABₙ₁: means a mixture of complexes of soluble native or recombinant TNF-α receptors p55 with the analogue TNF-α Cys95His107His108Cys148,
- ABₙ₂: means a mixture of complexes of soluble native or recombinant TNF-α receptors p75 with the analogue TNF-α Cys95His107His108Cys148, and thus
- ABₙ₁Bₙ₂: means a mixture of complexes of the analogue TNF-α Cys95His107His108Cys148 and of various truncated forms of the two native soluble TNF-α receptors p55 and p75.

One can speak about a mixture since it is well-known that at the N-terminal part of a soluble receptor proteolytic cleavage of several amino acids takes place and since on the same TNF-α trimer either of the soluble TNF-α receptors p55 or p75 may be bound.

Recombinant soluble TNF-α receptors p55 or p75 may be used instead of native soluble TNF-α receptors.

The recombinant analogue TNF-α Cys95His107His108Cys148 was chosen for immunization for the following reasons:
1. The double mutation His107His108 makes possible a sufficiently firm binding of the analogue onto the chromatographic column Cu2+--IDA and thus it serves as affinity ligand in one step isolation of soluble TNF receptors of natural origin (urine, plasma, pleural fluid etc.) as well as from cultures of recombinant organisms e.g. *E. coli*, yeast and insect or mammal cell cultures. The complex is eluted from the column by an elevated concentration of imidazole, i.e. in mild conditions which do neither adversely affect the biological properties of the complex or of the components thereof nor are they the cause of dissociation or denaturation of proteins.
2. The additional double mutation Cys95Cys148 makes possible a spontaneous linkage of subunits in the TNF trimer with disulfide bonds. Particular mutations Cys95 and Cys148 are present on interfaces which already by nature link the monomers into a rather compact trimer, which, however, according to hitherto known data at low concentrations (e.g. physiological concentrations) nevertheless dissociates into monomers and at the same time is denaturated and loses its biological action (US patent 5,223,395). By means of the double mutation of Cys 95Cys148 the undesired dissociation was prevented and the thermostability of the trimer was highly increased at the same time.
3. The introduction of the said mutations in no way affected the sites important for the binding of the receptors. This was concluded because the cytotoxicity in comparison with TNF-α did not change. The citotoxicity was determined on the L929 cell line.
4. The analogue TNF-α Cys95His107His108Cys148, besides being useful for a simple isolation of the receptors, also represents a compact, thermodynamically stable carrier nucleus for soluble receptors. Such a complex is more stable, more viable and also more immunogenic in comparison with the use of particular components separately.
5. Since the binding sites between TNF-α and the receptor are covered, only monoclonal antibodies directed to the uncovered surface areas may be formed with a greater probability, which is important at the use of said antibodies for analytical purposes (ELISA) and very important for a correct determination of TNF-α concentration in the presence of a large excess of soluble receptors.
6. For the preparation of monoclonal antibodies the isolation of individual pure receptors is not necessary. By the use of a mixture equivalent or even better results are achieved.

Some monoclonal antibodies of the present invention also exhibit a high neutralizing activity. They are used for therapeutic purposes. The remaining monoclonal antibodies make possible the determination of free macromolecules of TNF-α receptors p55, p75 and of TNF-α as well as of the mutual complexes thereof.

The measurements of the soluble TNF-α receptor p55 in serum by monoclonal antibodies of the present invention show e.g. that by means of these antibodies healthy volunteers, radically treated melanoma patients and metastatic melanoma patients may be differentiated. Also the success of chemoimmunotherapy treatment may be predicted thereby and the success of the treatment may be followed.

### EXAMPLES

The invention is illustrated by the following Examples but in no way limited thereto.

### Example 1

Preparation of the analogue TNF-α Cys95His107His108Cys148

### Gene and the expression system

A commercial synthetic TNF-α gene incorporated in the carrier plasmid BBG4 (British Biotechnology, Great Britain) was used. The gene had codons adapted for expression in bacterium *E. coli.*

Basic expression plasmid pCYTEXP1 (Belev T.N. et al., A fully modular vector system for optimization of gene expression in *Escherichia coli.* Plasmid 1991: **26:** 147-150) was supplied by the firm Medac (Hamburg, Germany). Enzymes used in our work were supplied by Sigma, Amershen, and Boehringer. When the source of reagents is not specifically mentioned, they are of a quality corresponding to the requirements for working in molecular biology.

The gene was cut out from the plasmid BBG4 by the pair of restrictases Ndel/BamHI and subcloned into the expression plasmid pCYTEXP1 that had been previously treated by the same pair of restrictases.

In this expression plasmid the TNF gene is controlled by thermoinducible promoter and hence the fermentation is to be performed at 40-42°C. In optimized fermentation conditions the expression of TNF-α in this system was about 1-2% of the whole cell proteins. Because of the relatively low expression the expression plasmid was modified in such a way that
a) the plasmid pCYTEXP1 was cut by the enzyme HindIII, the cohesive ends were filled in on both sides with Klenow enzyme and again ligated into a circular plasmid with T4 DNA ligase or
b) from the plasmid pCYTEXP1 a part of the repressor cI⁸⁵⁷ gene was cut out by a pair of enzymes HindIII/NotI, the ends were filled-in by Klenow enzyme and again ligated into a circular plasmid by T4 DNA ligase.

In both cases the plasmid DNA from ten clones was analysed. The selected clone had to have a correct size of the plasmid and a high expression already at 30°C. Thus an expression plasmid with a promoter that was not controlled by repressor cI⁸⁵⁷ any more was obtained and at the optimum run of the fermentation in a laboratory fermentor the expression of TNF-α was elevated to 15-25% of the whole cell proteins depending on the kind of the analogue. All TNF-α analogues mentioned herein were obtained by the expression plasmid thus modified. So it was shown that the constitutive expression of TNF-α or analogues thereof does not essentially affect the growth of *E. coli* and simultaneously a sufficiently large amount of the desired protein was accumulated in the. cytoplasm to make possible an economical isolation. Since the expression was not linked to temperature induction any more, the cells could also be cultivated at a lower temperature (e.g. at 30°C), which proved to be very favourable just in cystein mutants where at the usual induction temperature almost no accumulation of protein took place. The addition of various expensive substances (inductors e.g. IPTG) was omitted. Namely, in most commercial expression systems controlled promoters are used.

### Example 2

### The preparation of mutated TNF-α genes

The corresponding mutations were introduced by oligonucleotide directed mutagenesis on a single-strand plasmid DNA according to a well-known method (Kunkel, T.A, Rapid and efficient site specific mutagenesis without phenotypic selection. Proc. Natl. Acad.Sci. USA 82, 488-492) in two steps. Oligonucleotides were synthesised on a DNA synthetizer (model 381A) of the firm Applied Biosystems with chemicals supplied by the same firm.

First the starting analogue TNF-α His107His108 was prepared and for the introduction of mutations Glu107Gly108 → His107His108 there was used oligo M28z31 having the sequence

5'-CGC TTC TGC ATG GTG GGG AGT TTC ACG C-3'

In the next step both cystein mutations were introduced as well and for the mutation Ser95 → Cys95 there was used oligo M20z38 having the sequence 5'-CTT GAT AGC GCA CAG CAG GT-3' and for the mutation Gly148 → Cys148 there was used oligo M23z41 having the sequence

5'-GTA CAC CTG GCA AGA TTC AGC GA-3'

### Examples 3

### Isolation and purification of the analogue TNF-α Cys95His107His108Cys148

From fresh colonies of *E. coli* NM *522,* 10 to 30 ml of an overnight culture were prepared in a simple culture substrate LB with the addition of 100 µg of ampicillin/ml of the medium in Erlenmeyer flasks on the laboratory shaker at 30 °C and at 120-130 rpm. This culture was used as an inoculum for the preparation of biomass from a greater volume (1 - 3 1 of LB with the addition of ampicillin). The bacterial culture grew at the said conditions to the saturation phase (optical density at 550 nm was from 1 to 1.2). The cell pellet was separated from the culture substrate by centrifugation (5000 rpm; 5 minutes) and washed. The washed cells of the bacterial pellet were mechanically broken in Brown's MKS homogenizer. In the centrifugate a major part of nucleic acids was precipitated with 0.1% polyethyleneimine. Proteins were precipitated with ammonium sulfate (65% of saturation), the precipitate was dissolved in the buffer 0.02 M K-phosphate, 0.2 M NaCl, pH 7.1 and applied to a column of Zn⁺⁺-IDA Chelating Superose (Pharmacia). Also other ions e.g. Cu⁺⁺, Ni⁺⁺, Co⁺⁺ could be used. It was gradiently eluted with a buffer of the same composition and by the addition of imidazole (50 mM). Since our analogue had histidine mutations, it was retained on the column for the longest period of time with other proteins and the majority of other impurities being eluted earlier. Simultaneously with the biologically active analogue also a minor part of partially proteolytically degraded protein was eluted, which, however, could be eliminated in the phase of polishing on HIC column of Phenyl Superose (Pharmacia). The total yield at purification was 40-50%.

The purity and quality of the purified analogue was assessed by standard SDS-PAGE analysis (at silver staining only one major spot of the size 17 kDa was visible and there was barely visible a spot of a covalently linked TNF-α dimer non-reducible with mercaptoethanol). At performing SDS-PAGE without reducing agent - i.e. without mercaptoethanol - there was obtained only one spot in the height of 40-42 kDa corresponding to TNF-α trimer with all three subunits compactly joined by disulfide bonds. The isoelectric point (pI) was elevated to a value of about 7.85. One of the additonal properties of such a trimer was also an essentially increased thermostability.

Cytotoxicity test on standard L929 cell line of mouse fibroblasts showed that the biological activity was somewhat reduced but comparable to that of native TNF-α.

### Example 4

### Isolation of soluble receptors p55 and p75 from urine and preparation of complexes of the analogue TNF-α Cys95His107His108Cys148 and of soluble TNF-α receptors p55, p75

a) Preparation of protein fraction from urine
   From urine (501) of patients having various cancer diseases, a concentrate (600 ml) was obtained by ultrafiltration (membrane 10000 NMWL polysulfone cat. no. PTGC0MP04, apparatus Minitan, company Millipore). The proteins were precipitated with the addition of ammonium sulfate (up to 60% of saturation) and stored at -70°C until further treatment.
b) Isolation of receptors on an affinity column with immobilized analogue TNF-α Cys95His107His108Cys148
   The analogue TNF-α Cys95His107His108Cys148 (1 mg) dissolved in 1 ml of 0.02 M K-phosphate buffer with 0.2 M NaCl, pH 7.1 (buffer A), was bound onto an affinity column of Chelating Superose (Pharmacia) with immobilized Cu⁺⁺ prepared accoring to the manufacturer's instructions. On the column thus prepared ammonium sulfate precipitate of proteins from urine dissolved in buffer A was slowly applied for 30 minutes. Non-bound proteins were washed with buffer A. Then complexes between the analogue TNF-α Cys95His107His108Cys148 and soluble receptors as well as the excessive analogue TNF-α Cys95His107His108Cys148, which all simultaneously appeared in the same protein peak, were eluted by the linear gradient of imidazole (from 0 to 50 mM) in the buffer A. In the same way there was achieved the elution of the said protein complexes and of the excessive analogue TNF-α Cys95His107His108Cys148 by the gradient of histidine and glycine or only by lowering the pH of the starting buffer. In the case of elution by lowering the pH, the eluates had to be neutralized immediately after elution. The fractions containing protein complexes between the analogue TNF-α Cys95His107His108Cys148 and the soluble receptors as well as the excessive analogue TNF-α Cys95His107His108Cys148 were concentrated on Amicon membrane YM10.
c) Gel filtration
   The excessive analogue was separated from the complexes between the analogue TNF-α Cys95His107His108Cys148 and the soluble receptors by gel filtration on Superose 12 (Pharmacia) and Sephacryl 200 (Pharmacia) in the above buffer A, which was also used for the equilibration of the column.

### Example 5

### Immunization of mice

Groups of three BALB/c mice were immunized subcutaneously with a mixture of a complex of the analogue TNF-α Cys95His107His108Cys148 and soluble human TNF-α receptors p55 or p75. Both proteins were incubated for 45 minutes at room temperature and then blended with complete Freund's adjuvant (Sigma, St. Louis, MO, USA). After a month the mice were immunized intraperitoneally with the same doses of both proteins in incomplete Freund's adjuvant (Sigma, St. Louis, MO, USA). Ten days after the second inoculation mice has blood taken from the tail vein and the presence of polyclonal antibodies against both antigens in all sera was determined by the ELISA test. A month after the second immunization the mice having the best titre of specific antibodies were inoculated intravenously with the same doses of both complexes as in the first and the second inoculations, this time in a physiological solution. After three days the mice were sacrificed and their lymphoblasts were used for fusion.

### Example 6

### Preparation of stable hybridoma cell lines

Lymphocytes were isolated from the spleen of the sacrificed mice by perfusion and simultaneously also NS1 myeloma cells (>99% living cells) in the log phase of growth were prepared. Both kinds of cells were blended in 10:4 ratio and centrifuged. To the precipitate a 50% (v/v) solution of polyethylene glycole 1500 (PEG 1500; Sigma, St. Louis, MO, USA) in Eagle's medium modified according to Dulbecco (DMEM) was slowly added and then the suspension of cells was slowly diluted with the DMEM medium (1 ml during 1 minute and 20 ml during 5 minutes). The composition of the DMEM medium was as follows: DMEM (Flow, Scotland, Great Britain), 10 mM HEPES (Flow, Scotland, Great Britain) and 45 mM of NaHCO₃ (Sigma, St. Louis, MO, USA). After centrifugation the precipitate of cells was resuspended in a complete DMEM medium, which besides DMEM with the above composition also contained 13% of phoetal calf serum (Hy Clone, Utah, USA), 100 µg/ml of penicillin, 200 µg/ml of streptomycin and an addition of 2 mM of L-glutamine (Gibco, Scotland, Great Britain). As a nutrient substrate also a suspension of thymocytes prepared from thymuses of two-month BALB/c mice was added. The suspension of cells was given drop by drop into eight microtitre 96-well plates (Costar, Cambridge, MA, USA), 70 µl into each well. The plates were stored overnight at 37°C in an incubator saturated with steam and 5% CO₂. The next day the cells were fed with HAT DMEM (Sigma, St. Louis, MO, USA), 140 µl per well. Besides a complete DMEM medium, HAT DMEM also contained hypoxanthine (H), aminopterin (A) and thymidine (T). Only hybridomas grew in this medium whereas NS1 cells decayed in the presence of aminopterin and non-fused spleen cells had a limited lifetime in *in vitro* conditions. The plates were then all the time kept at 37°C in an incubator saturated with steam and 5% CO₂. HAT DMEM was fed every third day. On the fourth day the first clones of hybridomas could be observed. The supernatants were tested after seven days. Therein first the presence of mouse immunoglobulins was determined and then those supernatants giving very strong reactions in the ELISA test were tested also for the presence of antibodies against the analogue TNF-α Cys95His107His108Cys148 and recombinant human TNF-α. The hybridomas in the wells where in the supernatants specific antibodies having the greatest relative binding affinities had been determined, were cultured to greater volumes (at first in 24-well plates and then in bottles with gradually greater volumes). Thereat the complete HAT DMEM medium was first replaced by a complete HT DMEM medium and then by a complete DMEM medium.

To provide for the stability and homogeneity of the selected cell lines and for the antibodies produced by said lines to be actually monoclonal, a part of hybridomas was cloned by the limited dilution method and a part was frozen. The supernatants of the cloned hybridomas were tested again by the ELISA test. Selected hybridomas were cultured to greater volumes. One part thereof were frozen and another part was cultivated to maximum density. Such supernatants were used for the determination of the class and the subclass of immunoglobulins, for the determination of the relative binding affinity of monoclonal antibodies and for the determination of the concentrations of immunoglobulins in the exhausted supernatants of hybridomas.

### Example 7

### ELISA for the determination of mouse monoclonal antibodies against the analogue TNF-α Cys95His107His108Cys148, TNF-α as well as against p55 and p75 receptors

Microtitre plates with a flat bottom (Maxisorp, Nunc, Denmark) were coated with anti-mouse immunoglobulins (100 µl) (Dakopatts, Denmark) or the analogue TNF-α Cys95His107His108Cys148, TNF-α, p55 or p75 receptors in 50 mM of sodium carbonate/bicarbonate buffer, pH 9.6, in the concentration of 1 µg/ml. The plates were incubated overnight at 4°C. Then they were washed three times with isotonic phosphate buffer (PBS) with 0.05% Tween 20 (PBS/Tween₂₀, Sigma, St. Louis, MO, USA). The free sites were blocked by 100 µl of 1% bovine serum albumin (BSA) in PBS/Tween₂₀ for 1 hour at 37°C. After washing three times with PBS/Tween₂₀, 100 µl of supernatants of hybridomas were applied each time into the wells of a microtitre plate and incubated for 1 hour and 30 minutes at 37°C. The plates were then again washed with PBS/Tween₂₀ and onto each of them there were applied 100 µl of the conjugate (goat anti-mouse immunoglobulins conjugated with peroxidase; Jackson Immuno Research Laboratories, Dianova) diluted 1:5000 in 1% BSA-PBS/Tween₂₀. After the incubation for 1.5 hours at 37°C the plate was washed three times by an automatic plate washer and into each well 100 µl of a substrate [0.1% 2,2'-azinobis-(3-ethylbenzthiazoline sulfonic acid) (ABTS) and 0.012% H₂O₂ in a substrate buffer composed of 0.1 M citric acid and 0.1 M phosphate buffer, its pH being 4.5] were applied. After 30 minutes the absorbance at 410 nm was measured by an automatic plate reader. Also positive and negative controls were included into the test.

### Example 8

### ELISA for the quantitative determination of mouse IgG antibodies

The wells of a microtitre plate (Maxisorp, Nunc, Denmark) were coated with goat anti-mouse immunoglobulins (100 µl) (Dakopatts, Denmark) in 50 mM of sodium carbonate/bicarbonate buffer, pH 9.6, of the final concentration 1 µg/ml and incubated overnight at 4°C. After washing, blocking the plate with 1% BSA in PBS/Tween₂₀ and washing again three times with PBS/Tween₂₀, 100 µl of mouse immunoglobulins G or M of known concentrations (Sigma, St. Louis, MO, USA) as well as 100 µl of appropriately diluted supernatants of hybridomas, wherein the concentration of monoclonal antibodies was determined, were applied into each well. Another 100 µl of goat anti-mouse immunoglobulins conjugated by horseradish peroxidase (HRP) (Jackson Immuno Research Laboratories, Dianova) and diluted 1:5000 in 1% BSA-PBS/Tween₂₀ were applied to the plate after incubating for 1 hour and 30 minutes at 37°C and washing. After 1.5 hours of incubation at 37°C and washing, 100 µl of the substrate were added into each well. Its composition was already described in Example 7. After incubating for half an hour at 37°C, the absorbance was read at 410 nm by an automatic plate reader.

From the calibrating plot obtained with known concentrations of mouse immunoglobulins, the concentrations of monoclonal antibodies in the supernatants of hybridomas were calculated.

### Example 9

### Determination of the class and subclass of monoclonal antibodies

The classes and subclasses of mouse immunoglobulins were determined in two ways. One of them was ELISA which was the same as the ELISA described in Example 7 except that instead of test goat anti-mouse antibodies conjugated with peroxidase, there were used the optimum dilutions of specific immunoglobulins against mouse immunoglobulins M as well as against all four classes of mouse immunoglobulins G (IgG₁, IgG₂ₐ, IgG_{2b}, IgG₃) (Nordic, The Netherlands) in 1% BSA-PBS/Tween₂₀. Also positive and negative controls were included into the test. All samples were measured in duplicate.

The classes and subclasses of mouse immunoglobulins were also determined by the Sigma Immuno Type™ ISO-1 kit (Sigma, St. Louis, MO, USA) according to the manufacturer's instructions.

### Example 10

### Determination of the relative binding affinity of monoclonal antibodies

When the concentration of antibodies in supernatants of hybridomas was known or when already pure monoclonal antibodies were available, the relative binding affinity could be determined from the titration curve obtained in the indirect ELISA test described in Example 7 except that instead of the supernatants of hybridomas the dilutions thereof in 1% BSA-PBS/Tween₂₀ or dilutions of pure monoclonal antibodies were applied to a microtitre plate. The antibody concentration where the absorbance measured in ELISA test equalled the half-value of absorbance at saturation, gave - calculated in moles - the relative binding affinity of antibodies, which is a criterion for the affinity constant thereof.

Also positive and negative controls were included into the test. All samples were measured in duplicate.

### Example 11

### Purification of monoclonal antibodies

Monoclonal antibodies were purified on protein G-Sepharose (Sigma, St. Louis, MO, USA). Twenty-five times concentrated supernatants of hybridomas dialyzed in Centriprep 30 (Amicon, USA) against 0.02 M phosphate buffer, pH 8.0, which contained 0.5 M NaCl, were applied to a column of protein G-Sepharose. The sample was fastened for 30 minutes at room temperature. Then non-fastened proteins were washed with 0.02 M phosphate buffer, pH 8.0, which contained 0.5 M NaCl, and the bonded immunoglobulin fractions, i.e. monoclonal antibodies were eluted with 0.1 M glycine/HCl, pH 2.7. These fractions were immediately neutralized with 1 M Tris buffer, pH 9.0. The presence of antibodies in individual fractions was determined by measuring the absorbance at 280 nm and the activity thereof with an indirect ELISA test. The positive fractions were concentrated and dialyzed on Centriprep 30, then divided into aliquots and frozen to -20°C.

### Example 12

### Conjugation of monoclonal antibodies with horse radish peroxidase

Monoclonal antibodies were conjugated with HRP (type VI, Sigma, St. Louis, MO, USA) by means of a glutaraldehyde method (Antibodies, A laboratory manual, Harlow, E., Lane, D., editors, 1988).

HRP (2.9 mg) was dissolved in 1.25% glutaraldehyde/PBS (0.1 ml), pH 7.1, and incubated overnight at room temperature. Then the excessive glutaraldehyde was eliminated by filtration on Sephadex G-25 Superfine column equilibrated with 0.15 M NaCl. Brown-coloured fractions were collected and dialyzed against 0.1 M sodium carbonate/bicarbonate buffer, pH 9.2, and concentrated to 0.65 ml in Centriprep 30.

The selected pure monoclonal antibodies (1.5 mg) were dialyzed in Centricone (Amicon, USA) against 0.1 M sodium carbonate/bicarbonate buffer, pH 9.2, and concentrated to 0.25 ml, then the prepared HRP was added and it was incubated overnight at room temperature.

The next day 0.2 M lysine (0.1 ml) was added and it was incubated for 30 minutes at room temperature. The sample was dialyzed in Centricone against PBS, pH 7.1, and concentrated to 1 ml. Conjugated antibodies were stored in aliquots at -20°C.

### Example 13

### Optimization of the sandwich EUSA test for quantitative determination of TNF-α and its analogues (chessboard)

In order to optimize the ELISA test for the quantitative determination of TNF-α and its analogues, the wells on a microtitre plate with a flat bottom (Maxisorp, Nunc, Denmark) were coated with 100 µl of selected monoclonal antibodies of different dilutions (from 10 µg/ml to 80 ng/ml; dilutions 1:2ⁿ) in 50 mM sodium carbonate/bicarbonate buffer, pH 9.6. Into each of the eight lines the same dilutions of monoclonal antibodies were applied. The plates were incubated overnight at +4°C.

The next day they were washed three times with PBS/Tween₂₀. The free sites were blocked for 30 minutes with 100 µl of 1% BSA in PBS/Tween₂₀ at 37°C. Then 100 µl of TNF-α or of the analogue TNF-α Cys95His107His108Cys148 in 1% BSA-PBS/Tween₂₀ in the concentration of 0.5 µg/ml were applied into each well. After incubating for 1.5 hours at 37°C and repeated washing with PBS/Tween₂₀, 100 µl of different dilutions of the conjugate in 1% BSA-PBS/Tween₂₀ (from 1:50 to 1:6400; dilutions 1:2ⁿ) were applied into each of the eight columns of the microtitre plate. After incubating for 1.5 hours at 37°C and washing with PBS/Tween₂₀, 100 µl of the substrate ABTS (its composition was described in Example 7) were added into each well. After incubating for half an hour at 37°C, the absorbance at 410 nm was read by an automatic plate reader. Also positive and negative controls were included into the test.

### Example 14

### Calibrating plot for the determination of TNF-α and its analogues

The wells of a microtitre plate with a flat bottom (Maxisorp, Nunc, Denmark) were coated with an optimum concentration (1 µg/ml) of the selected monoclonal antibodies in 50 mM sodium carbonate/bicarbonate buffer, pH 9.6. After incubating overnight at +4°C, the plates were washed three times with PBS/Tween₂₀ and blocked for 30 minutes at 37°C with 1% BSA-PBS/Tween₂₀. After a repeated washing with PBS/Tween₂₀, 100 µl of different concentrations (from 10 µg/ml to 0.2 pg/ml) of TNF-α or its analogues in 1% BSA-PBS/Tween₂₀ were applied to each well. The plates were incubated for 1.5 hours at 37°C, washed three times with PBS/Tween₂₀ and 100 µl of an optimum dilution of the conjugate (1:1000), prepared as described in Example 12, in 1% BSA-PBS/Tween₂₀ were applied to each well. After incubating for 1.5 hours at 37°C and washing the plate with PBS/Tween₂₀, 100 µl of the substrate ABTS (the composition was given in Example 7) were added into each well. After incubating for half an hour at 37°C, the absorbance at 410 nm was read by an automatic plate reader.

### Example 15

### Optimization of the sandwich ELISA test for quantitative determination of bot soluble TNF-α receptors p55 and p75 (chessboard)

The chessboard was performed in the same way as disclosed for TNF-α or it analogue TNF-α Cys95His107His108Cys148. For either antigen two conjugates with horse radish peroxidase were prepared according to the method described in Example 12. Each conjugate was then tested in combination with four monoclona antibodied used for coating a microtitre plate, in order to find out which combination of monoclonal antibodies provided for the most sensitive ELISA test.

### Example 16

### Calibrating plots for the determination of soluble TNF-α receptors p55 and p75

The process and reagents were the same as at the calibrating plot for TNF-α and its analogue with the exception that the optimum concentration of monoclonal antibodies for coating the microtitre plate was 2 µg/ml and the optimum dilution of the conjugate was 1:1000.

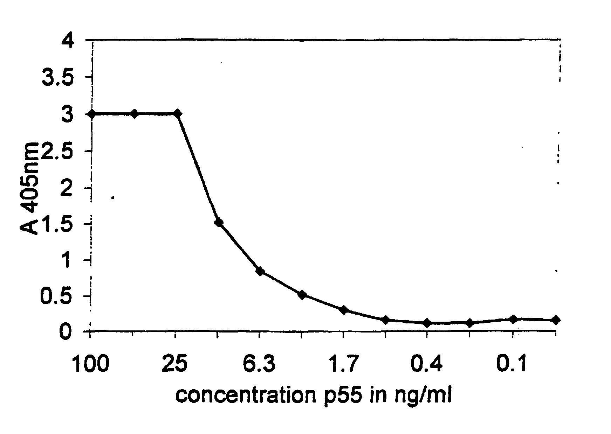

Fig. 1 represents the calibrating plot for the receptor p55.

### Example 17

### The determination of the soluble TNF-α receptor p55 in serum

The soluble TNF-α receptor p55 was determined in the serum of 69 healthy volunteers, in 35 patients having malignant melanoma and having been without signs of the disease for at least 30 months since the primary surgery treatment and in 47 patients having metastatic melanoma.

Healthy blood donors were included into the control group. They had been healthy at least 14 days before having their blood taken. Thus they had not suffered any respiratory or other infections. No infective liver disease or HIV virus infection had been found in any of them. Their mean age was 21 years (19 to 25).

The group with malignant melanoma without signs of the disease consisted of 31 patients, 15 men and 16 women, of the mean age of 46 years (17 to 73). None of them had shown any signs of the disease for at least 3 years since the primary melanoma surgery. In the preceding 6 months no one had been treated for chronic degenerative diseases of joints or liver disease, hypertension, diabetes or hearth diseases. Fourteen days before having their blood taken no one had shown any signs of urinary or respiratory infections.

The group with metastatic melanoma consisted of 47 patients, 19 men and 28 women, of the mean age of 47 years (18 to 66). Twenty-one patients were in capacity state 0 according to WHO, 15 patients in state 1 and 9 patients in state 2. 21 patients had one metastatic location, 17 patients had 2 metastatic locations and 9 patients had 3 or more metastatic locations. 22 patiens had skin metastases, 21 patients had metastases in the lymphatic glands, 12 in the lung, 4 in bones and 4 in liver. No one of these patients had been treated for chronic diseases. At least 2 months before having their blood taken for the determination of the soluble TNF-α receptor p55, they had not been treated by chemotherapy and biological response modifiers. Their blood was taken one day before the beginning of treatment by chemoimmunotherapy, on day 57 and on day 100 to 120 from the beginning of the treatment.

The patients received chemoimmunotherapy according to the following scheme: Vinblastin 2 mg/m² of the body surface in a 12-hour infusion, lomustin 60 mg/m² p. o. on the first day, cisplatin 20 mg/m² in a 2-hour infusion on the 2nd, the 3rd, the 4th, the 5th day, and interferon alpha 2b 6 MIE subcutaneously on the 3rd to the 7th day. The cycle was repeated every 28 days.

The second blood-taking for the determination of the soluble TNF-α receptor p55 was on day 57 from the beginning of the treatment i.e. before the third cycle. The third blood-taking was three weeks after the fifth cycle of the treatment, i.e. on day 100 to 120 after the beginning of the treatment.

The mean value of the receptor p55 in the serum and 95% Confidence interval CI were determined in each group.

The values of soluble TNF-α receptor p55 in healthy volunteers, in melanoma patients treated radically, and in patients with metastatic melanoma are presented in Table 1.

**TABLE 1**

| | Control group | Patients without the disease | Metastatic melanoma |
|---|---|---|---|
| | | | |
| Number of patients | 69 | 31 | 47 |
| | | | |
| Mean value | 0.50 | 0.37 | 2.1 |
| (95% CI) | (0.28-0.72) | (0.21-0.52) | (0.37-3.82) |
| | | | |
| Lowest value (95% CI) | 0.05 | 0.1 | 0.1 |
| | | | |
| Highest value (95% CI) | 5.00* | 2.00 | 27.00 |
| | | | |
| Standard deviation | 0.91 | 0.42 | 5.86 |

| | | | |
|---|---|---|---|
| * Six volunteers had the value for the soluble TNF-α receptor p55 higher than 2.00. The reason for such high values was not investigated. For the remaining 63 volunteers the mean value of p55 was 0.24, confidence interval 0.17 to 0.31 and the maximum value 2.00. | | | |

In Table 2 the values of the soluble TNF-α receptor p55 after two chemotherapy cycles for the patients with metastatic melanoma of the Table 1 are given. They are divided into two subgroups with regard to the response to treatment. The patients with a low value of the soluble TNF-α receptor p55 responded well to the therapy whereas the patients with a high value did not. Similar values were also determined before the beginning of the third chemoimmunotherapy cycle or after the fifth (last) cycle.

**TABLE 2**

| | Response to chemotherapy | No response to chemotherapy |
|---|---|---|
| | | |
| Number of patients | 18 | 29 |
| | | |
| Mean value | 0.16 | 3.3 |
| | | |
| Lowest value | 0.1 | 0.1 |
| | | |
| Highest value | 0.4 | 27.00 |
| | | |
| Standard deviation | 0.10 | 7.26 |
| | | |
| 95% CI | 0.11-0.21 | 0.54-6.10 |

### Example 18

### Pharmaceutical preparations with monoclonal antibodies

Pharmaceutical preparations contain a neutralizing monoclonal anti-TNF-α antibody. The preparations may be in a form suitable for parenteral application, i.e. in the form of injections or lyophilized powder, which by the addition of an appropriate solution, e.g. water for injections gives an injection solution. The amount of antibodies in the preparations depends upon the purpose of use, the application route, the age and the state of the patient. Generally, the preparations contain so many anti-TNF-α antibodies that by a single application 0.1 to 5 mg anti-TNF-α per kg of body weight may be administered. The preparations contain either anti-TNF-α antibodies alone or with the addition of various antihistaminics and/or glucocorticoids. Additionally, various substances for isotonization, solubilization, stabilization and pH regulation are added to the preparations.

As an example the following composition of a preparation is given:

| | |
|---|---|
| anti-TNF-α | 50.00 mg |
| sodium chloride | 112.00 mg |
| sodium primary phosphate | 11.25 mg |
| sodium secondary phosphate | 45.00 mg |
| polysorbate 80 | 5.00 mg |
| water for injections | ad 5 ml |

### Information about the deposit of hybridoma cell lines

Hybridoma cell lines marked C9/8, C7/12 and B12/1, are deposited in the European Collection of Cell Cultures under Nos. 95101016, 95071217 and 95071218.

## Claims

1. Monoclonal antibodies and fragments thereof which are capable to bind either to the available surface area of human TNF-α or TNF-α analogues or free soluble TNF-α receptors p55 or p75 as well as of complexes thereof, **characterized in that** they are obtained by cultivating a stable hybridoma cell line ECACC 95101016.

2. Monoclonal antibodies and fragments thereof which are capable to bind either to the available surface area of human TNF-α or TNF-α analogues or free soluble TNF-α receptors p55 or p75 as well as of complexes thereof, **characterized in that** they are obtained by cultivating a stable hybridoma cell line ECACC 95071217.

3. Monoclonal antibodies and fragments thereof which are capable to bind either to the available surface area of human TNF-α or TNF-α analogues or free soluble TNF-α receptors p55 or p75 as well as of complexes thereof, **characterized in that** they are obtained by cultivating a stable hybridoma cell line ECACC 95071218.

4. Stable hybridoma cell line ECACC 95101016.

5. Stable hybridoma cell line ECACC 95071217.

6. Stable hybridoma cell line ECACC 95071218.

7. A process for the preparation of a stable hybridoma cell line according to claims 4 to 6, **characterized in that** it comprises
- an immunization of mice with the mixture of a complex of the TNF-*α* analogue Cys95His107His108Cys148 and of soluble native or recombinant TNF-α receptors p55 and/or p75,
- a fusion of the isolated mouse spleen cells with a cell line of myeloma cells, and
- cloning of hybridomas producing monoclonal antibodies.

8. The process according to claim 7, **characterized in that** for the immunization a complex of the TNF-α analogue Cys95His107His108Cys148 and of the soluble native or recombinant TNF-α receptor p55 is used.

9. The process according to claim 7, **characterized in that** for the immunization a complex of the TNF-α analogue Cys95His107His108Cys148 and of the soluble native or recombinant TNF-α receptor p75 is used.

10. The process according to claim 7, **characterized in that** for the immunization a complex of the TNF-α analogue Cys95His107His108Cys148 and of both soluble native or recombinant TNF-α receptors p55 and p75 is used.

11. Use of monoclonal antibodies and fragments thereof according to claim 1 for preparing a test kit for the determination of human TNF-α, free and complexed with soluble TNF-α receptors p55 and p75, in biological samples.

12. Use of monoclonal antibodies and fragments thereof according to claim 2 for preparing a test kit for the determination of human soluble TNF-α receptor p55, free and complexed with TNF-α, in biological samples.

13. Use of monoclonal antibodies and fragments thereof according to claim 3 for preparing a test kit for the determination of human soluble TNF-α receptor p75, free and complexed, in biological samples.

14. A method for a quantitative determination of human TNF-α, free and complexed with soluble TNF-α receptors p55 or p75 in biological samples by using monoclonal antibodies or fragments thereof according to claim 1.

15. A method for a quantitative determination of TNF-α receptors p55 free and complexed with TNF-α, in biological samples by using monoclonal antibodies or fragments thereof according to claim 2.

16. A method for a quantitative determination of TNF-α receptors p75 free and complexed with TNF-α, in biological samples by using monoclonal antibodies or fragments thereof according to claim 3.

17. Kit for the determination of the human TNF-α free and complexed with soluble TNF-α receptors p55 and/or p75, in biological samples, **characterized in that** it contains monoclonal antibodies and fragments thereof according to claim 1.

18. Kit for the determination of the soluble TNF-α receptor p55, free and complexed with TNF-α, in biological samples, **characterized in that** it contains monoclonal antibodies and fragments thereof according to claim 2.

19. Kit for the determination of the soluble TNF-α receptor p75, free and complexed with TNF-α, in biological samples, **characterized in that** it contains monoclonal antibodies and fragments thereof according to claim 3.

20. TNF-α analogue Cys95His107His108Cys148.

21. A process for preparing the TNF-α analogue according to claim 20, **characterized in that** it comprises
- expression in a recombinant *E. coli* strain and
- one-step purification by chelate chromatography.

22. Use of the TNF-α analogue according to claim 20 for the isolation of TNF-α soluble receptors p55 and p75 from human urine.

## Patentansprüche

1. Monoklonale Antikörper und deren Fragmente, die fähig sind, sich an die verfügbare Oberfläche entweder des humanen TNF-α oder der TNF-α-Analoga oder freier löslicher TNF-α-Rezeptoren p55 oder p75 sowie deren Komplexe zu binden, **dadurch gekennzeichnet, dass** sie durch Kultivierung einer stabilen Hybridoma-Zell-Linie ECACC 95101016 erhalten werden.

2. Monoklonale Antikörper und deren Fragmente, die fähig sind, sich an die verfügbare Oberfläche entweder des humanen TNF-α oder der TNF-α-Analoge oder freier löslicher TNF-α-Rezeptoren p55 oder p75 sowie deren Komplexe zu binden, **dadurch gekennzeichnet, dass** sie durch Kultivierung einer stabilen Hybridoma-Zell-Linie ECACC 95071217 erhalten werden.

3. Monoklonale Antikörper und deren Fragmente, die fähig sind, sich an die verfügbare Oberfläche entweder des humanen TNF-α oder der TNF-α-Analoge oder freier löslicher TNF-α-Rezeptoren p55 oder p75 sowie deren Komplexe zu binden, **dadurch gekennzeichnet, dass** sie durch Kultivierung einer stabilen Hybridoma-Zell-Linie ECACC 95071218 erhalten werden.

4. Stabile Hybridoma-Zell-Linie ECACC 95101016.

5. Stabile Hybridoma-Zell-Linie ECACC 95071217.

6. Stabile Hybridoma-Zell-Linie ECACC 95071218.

7. Verfahren zur Herstellung der stabilen Hybridoma-Zell-Linie nach Ansprüchen 4 bis 6, **dadurch gekennzeichnet, dass** es
- Immunisierung der Mäuse mit einem Gemisch aus einem Komplex des TNF-α-Analogs Cys95His107His108Cys148 und löslicher nativer oder rekombinanter TNF-α-Rezeptoren p55 und/oder p75,
- eine Fusion der isolierten Mausemilzzellen mit einer Zell-Linie der Myelomazellen, und
- Klonierung von monoklonale Antikörper produzierenden Hybridomen umfasst.

8. Verfahren nach Anspruch 7, **dadurch gekennzeichnet, dass** zur Immunisation ein Komplex des TNF-α-Analogs Cys95His107His108Cys148 und des löslichen nativen oder rekombinanten TNF-α-Rezeptors p55 verwendet wird.

9. Verfahren nach Anspruch 7, **dadurch gekennzeichnet, dass** zur Immunisation ein Komplex des TNF-α-Analogs Cys95His107His108Cys148 und des löslichen nativen oder rekombinanten TNF-α-Rezeptors p75 verwendet wird.

10. Verfahren nach Anspruch 7, **dadurch gekennzeichnet, dass** zur Immunisation ein Komplex des TNF-α-Analogs Cys95His107His108Cys148 und der beiden löslichen nativen oder rekombinanten TNF-α-Rezeptoren p55 und p75 verwendet wird.

11. Verwendung von monoklonalen Antikörpern und deren Fragmenten nach Anspruch 1 zur Herstellung eines Testkits zur Bestimmung von humanem TNF-α, frei und mit löslichen TNF-α-Rezeptorem p55 und p75 komplexiert, in biologischen Proben.

12. Verwendung von monoklonalen Antikörpern und deren Fragmenten nach Anspruch 2 zur Herstellung eines Testkits zur Bestimmung von humanem löslichem TNF-α-Rezeptor p55, frei und mit TNF-α komplexiert, in biologischen Proben.

13. Verwendung von monoklonalen Antikörpern und deren Fragmenten nach Anspruch 3 zur Herstellung eines Testkits zur Bestimmung von humanem löslichem TNF-α-Rezeptor p75, frei und komplexiert, in biologischen Proben.

14. Methode zur quantitativen Bestimmung von humanem TNF-α, frei und mit löslichen TNF-α-Rezeptorem p55 oder p75 komplexiert, in biologischen Proben unter Verwendung von monoklonalen Antikörpern oder deren Fragmenten nach Anspruch 1.

15. Methode zur quantitativen Bestimmung von TNF-α-Rezeptoren p55, frei und mit TNF-α komplexiert, in biologischen Proben unter Verwendung von monoklonalen Antikörpern oder deren Fragmenten nach Anspruch 2.

16. Methode zur quantitativen Bestimmung von TNF-α-Rezeptoren p75, frei und mit TNF-α komplexiert, in biologischen Proben unter Verwendung von monoklonalen Antikörpern oder deren Fragmenten nach Anspruch 3.

17. Kit zur Bestimmung von humanem TNF-α, frei und mit löslichen TNF-α-Rezeptoren p55 und/oder p75 komplexiert, in biologischen Proben, **dadurch gekennzeichnet, dass** es monoklonale Antikörper und deren Fragmente nach Anspruch 1 enthält.

18. Kit zur Bestimmung von löslichem TNF-α-Rezeptor p55, frei und mit TNF-α komplexiert, in biologischen Proben, **dadurch gekennzeichnet, dass** es monoklonale Antikörper oder deren Fragmente nach Anspruch 2 enthält.

19. Kit zur Bestimmung von löslichem TNF-α-Rezeptor p75, frei und mit TNF-α komplexiert, in biologischen Proben, **dadurch gekennzeichnet, dass** es monoklonale Antikörper oder deren Fragmente nach Anspruch 3 enthält.

20. TNF-α-Analogon Cys95His107His108Cys148.

21. Verfahren zur Herstellung vom TNF-α-Analogon nach Anspruch 20, **dadurch gekennzeichnet, dass** es
- eine Expression in rekombinantem *E. coli*-Stamm und
- eine einstufige Reinigung mittels Chelatenchromatographie umfasst.

22. Verwendung vom TNF-α-Analogon nach Anspruch 20 zur Isolierung von TNF-α löslichen Rezeptoren p55 und p75 aus humanem Harn.

## Revendications

1. Anticorps monoclonaux et leurs fragments, qui sont capables de se lier à la surface disponible soit du TNF-α humain soit d'analogues de TNF-α ou de récepteurs de TNF-α solubles libres p55 ou p75 et également de leurs complexes, **caractérisés en ce qu'**ils sont obtenus par culture d'une lignée cellulaire d'hybridome stable ECACC 95101016.

2. Anticorps monoclonaux et leurs fragments, qui sont capables de se lier à la surface disponible soit du TNF-α humain soit d'analogues de TNF-α ou de récepteurs de TNF-α solubles libres p55 ou p75 et également de leurs complexes, **caractérisés en ce qu'**ils sont obtenus par culture d'une lignée cellulaire d'hybridome stable ECACC 95071217.

3. Anticorps monoclonaux et leurs fragments, qui sont capables de se lier à la surface disponible soit du TNF-α humain soit d'analogues de TNF-α ou de récepteurs de TNF-α solubles libres p55 ou p75 et également de leurs complexes, **caractérisés en ce qu'**ils sont obtenus par culture d'une lignée cellulaire d'hybridome stable ECACC 95071218.

4. Lignée cellulaire d'hybridome stable ECACC 95101016.

5. Lignée cellulaire d'hybridome stable ECACC 95071217.

6. Lignée cellulaire d'hybridome stable ECACC 95071218.

7. Procédé pour la préparation d'une lignée cellulaire d'hybridome stable suivant les revendications 4 à 6, **caractérisé en ce qu'**il comprend
- une immunisation de souris avec le mélange d'un complexe de l'analogue de TNF-α Cys95His107His108Cys148 et des récepteurs de TNF-α naturels ou recombinants solubles p55 et/ou p75,
- une fusion de cellules spléniques isolées de souris avec une lignée cellulaire de cellules de myélome, et
- un clonage des hybridomes producteurs d'anticorps monoclonaux.

8. Procédé suivant la revendication 7, **caractérisé en ce que**, pour l'immunisation, un complexe de l'analogue de TNF-α Cys95His107His108Cys148 et du récepteur de TNF-α naturel ou recombinant soluble p55 est utilisé.

9. Procédé suivant la revendication 7, **caractérisé en ce que**, pour l'immunisation, un complexe de l'analogue de TNF-α Cys95His107His108Cys148 et du récepteur de TNF-α naturel ou recombinant soluble p75 est utilisé.

10. Procédé suivant la revendication 7, **caractérisé en ce que**, pour l'immunisation, un complexe de l'analogue de TNF-α Cys95His107His108Cys148 et des deux récepteurs de TNF-α naturels ou recombinants solubles p55 et p75 est utilisé.

11. Utilisation d'anticorps monoclonaux et de leurs fragments suivant la revendication 1, pour la préparation d'un kit analytique pour la détermination du TNF-α humain, libre et complexé avec les récepteurs de TNF-α solubles p55 et p75, dans des échantillons biologiques.

12. Utilisation d'anticorps monoclonaux et de leurs fragments suivant la revendication 2, pour la préparation d'un kit analytique destiné à la détermination du récepteur de TNF-α humain soluble p55, libre et complexé avec le TNF-α, dans des échantillons biologiques.

13. Utilisation d'anticorps monoclonaux et de leurs fragments suivant la revendication 3, pour la préparation d'un kit analytique destiné à la détermination du récepteur de TNF-α humain soluble p75, libre et complexé, dans des échantillons biologiques.

14. Méthode pour une détermination quantitative du TNF-α humain, libre et complexé avec les récepteurs de TNF-α solubles p55 ou p75 dans des échantillons biologiques en utilisant des anticorps monoclonaux ou leurs fragments suivant la revendication 1.

15. Méthode pour une détermination quantitative des récepteurs de TNF-α p55 libres et complexés avec le TNF-α, dans des échantillons biologiques, en utilisant des anticorps monoclonaux ou leurs fragments suivant la revendication 2.

16. Méthode pour la détermination quantitative des récepteurs de TNF-α p75 libres et complexés avec le TNF-α, dans des échantillons biologiques, en utilisant des anticorps monoclonaux ou leurs fragments suivant la revendication 3.

17. Kit pour la détermination du TNF-α humain libre et complexé avec les récepteurs de TNF-α solubles p55 et/ou p75, dans des échantillons biologiques, **caractérisé en ce qu'**il contient des anticorps monoclonaux et leurs fragments suivant la revendication 1.

18. Kit pour la détermination du récepteur de TNF-α soluble p55, libre et complexé avec le TNF-α, dans des échantillons biologiques, **caractérisé en ce qu'**il contient des anticorps monoclonaux et leurs fragments suivant la revendication 2.

19. Kit pour la détermination du récepteur de TNF-α soluble p75, libre et complexé avec le TNF-α, dans des échantillons biologiques, **caractérisé en ce qu'**il contient des anticorps monoclonaux et leurs fragments suivant la revendication 3.

20. Analogue de TNF-α Cys95His107His108Cys148.

21. Procédé pour la préparation de l'analogue de TNF-α suivant la revendication 20, **caractérisé en ce qu'**il comprend
- l'expression dans une souche de *E. coli* recombinante, et
- une purification en une étape par chromatographie avec un chélate.

22. Utilisation de l'analogue de TNF-α suivant la revendication 20, pour l'isolement des récepteurs de TNF-α solubles p55 et p75 à partir d'urine humaine.
